# EUROPEAN PATENT APPLICATION

(11) **EP 4 545 632 A1**
(43) Date of publication of application: **30.04.2025**
(21) Application number: 23831400.9
(22) Date of filing: 26.06.2023
(51) Int. Cl.: C12N 5/0783, A61K 35/15, A61K 35/17, A61K 35/545, A61P 35/00

(54) **CELL CULTURE METHOD**

(30) Priority: 27.06.2022 JP 2022102986
(71) Applicant: Takeda Pharmaceutical Company Limited, Osaka-shi, Osaka 541-0045 (JP)
(72) Inventor: KUWAE, Shinobu, Fujisawa-shi, Kanagawa 251-0012 (JP); MATSUNO, Keigo, Fujisawa-shi, Kanagawa 251-0012 (JP)
(74) Representative: Jones, Nicholas Andrew
(86) International application number: PCT/JP2023/023664
(87) International publication number: WO 2024/004960

(57) **Abstract**

The present invention aims to provide a method that makes it possible to sufficiently proliferate cells, particularly immunocompetent cells, even in a serum-free medium or low-serum medium, and a medium additive and a medium composition to be used for the method. An effect of maintaining and promoting cell proliferation comparable to that achieved by culture in a serum-containing medium can be obtained even in a serum-free medium or a low-serum medium, by containing boric acid or a salt thereof.

## Description

### [Technical Field]

The present invention relates to a method for culturing/proliferating cells in a serum-free medium or low-serum medium, and a medium additive and a medium composition for said method. The present invention relates to a method for producing a cell population in which the number of desired cells is expanded in a serum-free or low-serum medium, and a medicament containing the cell population obtained by said method.

### (Background of the Invention)

Conventionally, cell culture has been performed using a medium containing serum (hereinafter also to be referred to as "serum-containing medium"). For example, fetal bovine serum (FBS) is widely used in cell culture as an important additive for cell proliferation. However, when cultured cells or derivatives thereof are used for medical purposes, xenogeneic components may become a source of infection for blood-borne pathogens or heterologous antigens. In addition, differences between serum lots may cause variations in culture results. For this reason, in recent years, it has become mainstream to reduce as much as possible the amount of serum to be used and to culture cells using a medium with a clear chemical composition (chemically defined medium), and the development of serum-free medium and low-serum medium is underway.

Recently, chimeric antigen receptor (CAR) T cell (hereinafter to be simply referred to as CAR-T cell) therapy has been developed as one of the immunotherapies for cancer patients, in which the T cell receptor (TCR) of cytotoxic T cells (CTL) is genetically modified to make CTL directly and selectively recognize tumor cells, and an antitumor effect is exerted. Cancer therapy using CAR-T cells kills cancer cells based on a mechanism different from that of conventional anticancer drugs and radiation therapy and is therefore expected to be effective against intractable or treatment-resistant cancers. Current CAR-T cell therapy is generally performed using autologous CAR-T cell therapy, in which CAR genes are introduced ex vivo into T cells collected from a patient, and the CAR-T cells produced thereby are then administered to the patient, as in Kymriah (trade name) and Yescarta (trade name) approved in the United States. However, this method requires multiple steps over a long period of time, such as activation/proliferation of T cells, preparation of viral vectors, and gene transfer into T cells, and therefore has the problem of high manufacturing costs due to the costs required for cell culture and preparation of viral vectors. Therefore, iPS cell-derived CAR-T cells that enable allogeneic CAR-T therapy have been developed. In this method, too, a serum-containing medium is used in the process of producing iPS cell-derived CAR-T cells, particularly in the expansion culture process, in order to achieve a sufficient cell proliferation effect. However, from the viewpoint of GMP (Good Manufacturing Practice), the medium is preferably xeno-free, i.e., not containing xenogeneic components, and desirably serum-free or low-serum. In addition, considering that T cells derived from iPS cells are particularly vulnerable to washing stress compared to primary T cells, there has been a strong demand for a serum-free or low-serum medium that does not require extensive washing.

There have been many reports on methods for maintaining desired cell proliferation effects or promoting cell proliferation effects in cell culture. For example, there is also a report on the cell proliferation effect of boric acid via its transporter NaBC1 (Non Patent Literature 1). However, the medium used here is a serum-containing medium, and it is unclear whether the same cell proliferation effect is achieved in a serum-free medium or a low-serum medium. Patent Literature 1 describes a serum-free medium containing borate and suitable for culturing diploid cells. In addition, Non Patent Literature 2 describes that Taut, which is a taurine transporter, is important for the recall response of T cells.

### [Citation List]

### [Patent Literature]

[Patent Literature 1]
Chinese Patent No. 105462912

### [Non Patent Literature]

[Non Patent Literature 1]
   Park, Meeyoung et al., Mol Cell. 2004 Nov 5; 16(3):331-41.
[Non Patent Literature 2]
   Kaesler, Susanne et al., Eur. J. Immunol. 2012 42:831-841

### [Summary of Invention]

### [Technical Problem]

The present invention aims to provide a method for culturing and sufficiently proliferating cells, particularly immunocompetent cells, in a serum-free medium or low-serum medium, and a medium additive and a medium composition to be used for the method. Furthermore, the present invention aims to provide a method for producing a cell population with an expanded number of desired cells, particularly immunocompetent cells, in a serum-free medium or low-serum medium, and a medicament containing a cell population obtained by said method.

### [Solution to Problem]

In view of the above-mentioned problems, the present inventors prepared various factors to be added to a serum-free or low-serum medium and investigated their effects on cell culture or proliferation. As a result, an effect of maintaining and promoting cell proliferation comparable to that achieved by culture in a serum-containing medium was confirmed even when a serum-free medium or a low-serum medium was used, by adding boric acid or a salt thereof. Based on these findings, they have found more preferable culture conditions and completed the present invention.

Accordingly, the present invention provides the following.
[1] A method for culturing a cell, comprising a step of culturing the cell in a serum-free medium or low-serum medium comprising boric acid or a salt thereof.
[2] The method of the above-mentioned [1], wherein the final concentration of the boric acid or a salt thereof in the medium is 0.1 to 0.9 mM.
[3] The method of the above-mentioned [1], wherein the final concentration of the boric acid or a salt thereof in the medium is 0.3 to 0.6 mM.
[4] The method of any of the above-mentioned [1] to [3],
   wherein the aforementioned serum-free medium or low-serum medium is a serum-free medium.
[5] The method of any of the above-mentioned [1] to [4], wherein the cell is an immunocompetent cell.
[6] The method of the above-mentioned [5], wherein the immunocompetent cell is selected from dendritic cell, B cell, T cell, and natural killer cell.
[7] The method of the above-mentioned [6], wherein the immunocompetent cell is a T cell.
[8] The method of any of the above-mentioned [1] to [7], wherein the cell is derived from a pluripotent stem cell.
[9] The method of the above-mentioned [8], wherein the pluripotent stem cell is an iPS cell.
[10] The method of any of the above-mentioned [1] to [9], wherein the aforementioned medium further comprises taurine.
[11] A method for proliferating a cell, comprising a step of culturing the cell in a serum-free medium or low-serum medium comprising boric acid or a salt thereof.
[12] The method of the above-mentioned [11], wherein the aforementioned serum-free medium or low-serum medium is a serum-free medium.
[13] The method of the above-mentioned [11] or [12], wherein the aforementioned medium further comprises taurine.
[14] A method for producing a cell population in which the number of desired cells is expanded, comprising a step of culturing a cell population comprising the desired cells in a serum-free medium or low-serum medium comprising boric acid or a salt thereof.
[15] The method of the above-mentioned [14], wherein the aforementioned serum-free medium or low-serum medium is a serum-free medium.
[16] The method of the above-mentioned [14] or [15], wherein the aforementioned medium further comprises taurine.
[17] A medicament comprising a cell population obtained by the method of any of the above-mentioned [14] to [16].
[18] The medicament of the above-mentioned [17], which is for use in the prophylaxis and/or treatment of cancer.
[19] A medium additive comprising boric acid or a salt thereof, wherein the medium is a serum-free medium or a low-serum medium.
[20] The additive of the above-mentioned [19], wherein the aforementioned serum-free medium or low-serum medium is a serum-free medium.
[21] The additive of the above-mentioned [19] or [20], wherein the aforementioned medium further comprises taurine.
[22] A medium composition comprising boric acid or a salt thereof, wherein the composition is obtained by adding the medium additive of any of the above-mentioned [19] to [21] to a serum-free medium or low-serum medium.
[23] The medium composition of the above-mentioned [22], wherein the aforementioned serum-free medium or low-serum medium is a serum-free medium.
[24] The medium composition of the above-mentioned [22], further comprising taurine.
[25] A method for the prophylaxis and/or treatment of cancer, comprising administering a cell population obtained by the method of any of the above-mentioned [14] to [16] to a subject in need thereof.
[26] A cell population obtained by the method of any of the above-mentioned [14] to [16], which is for use in the prophylaxis and/or treatment of cancer.
[27] Use of a cell population obtained by the method of any of the above-mentioned [14] to [16] in the production of a medicament for the prophylaxis and/or treatment of cancer.

### [Advantageous Effects of Invention]

According to the present invention, a cell proliferation maintaining-promoting effect comparable to that by culture in a serum-containing medium can be obtained even when a serum-free medium or low-serum medium is used. In particular, culture in a serum-free medium or low-serum medium that does not require extensive washing is preferred for cells that are vulnerable to a stress caused by operations such as washing of cell and the like, and the cells are suitable for culture under such conditions.

### [Brief Description of Drawings]

[Fig. 1]

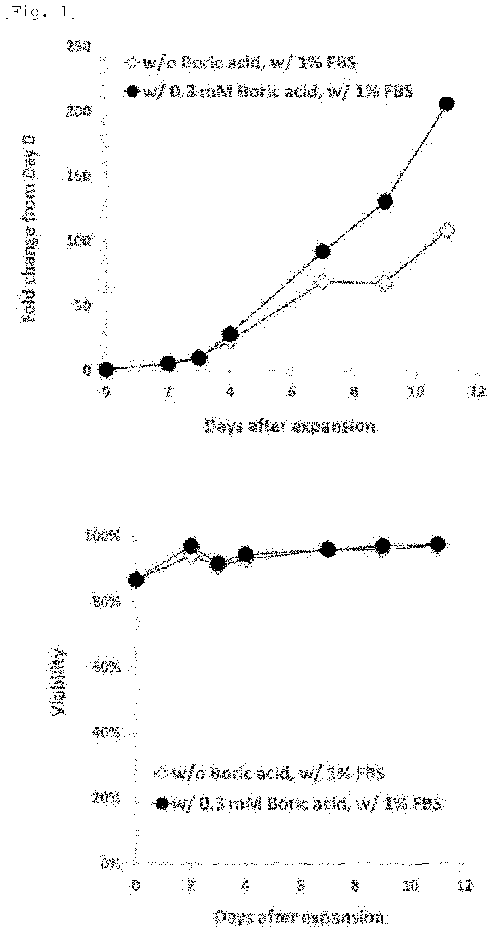

   Fig. 1 is a graph showing the results of a cell proliferation test of iPS cell-derived CAR-T cells that verify the effect of adding 0.3 mM boric acid to a 1% FBS-containing medium. The upper row shows the cell proliferation fold from day 0 of expansion culture, and the lower row shows the measurement results of the cell viability. w/o; without, w/; with
[Fig. 2]

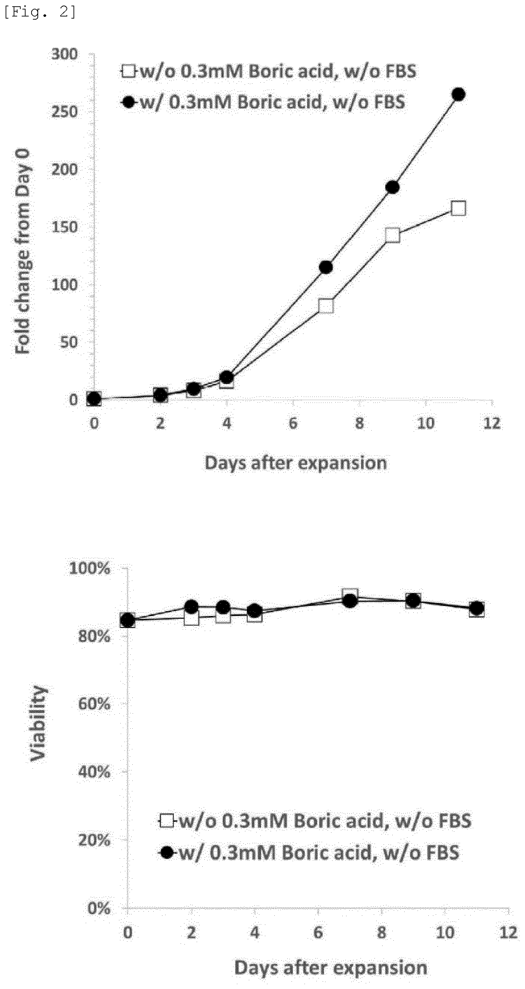

   Fig. 2 is a graph showing the results of a cell proliferation test of iPS cell-derived CAR-T cells that verify the effect of adding 0.3 mM boric acid to a serum-free expansion culture medium. The upper row shows the cell proliferation fold from day 0 of expansion culture, and the lower row shows the measurement results of the cell viability. w/o; without, w/; with
[Fig. 3]

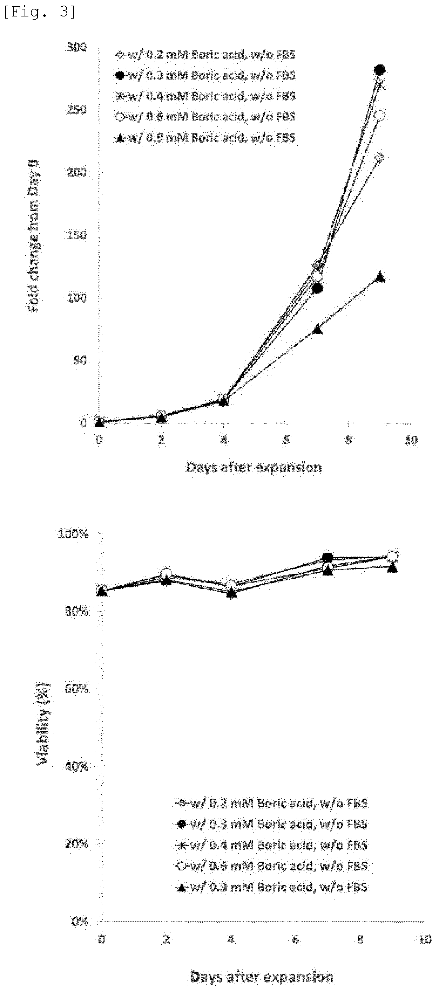

   Fig. 3 is a graph showing the results of a proliferation test of iPS cell-derived CAR-T cells when boric acid was added at different concentrations to a serum-free expansion culture medium. The upper row shows the cell proliferation fold from day 0 of expansion culture, and the lower row shows the measurement results of the cell viability. w/o; without, w/; with
[Fig. 4]

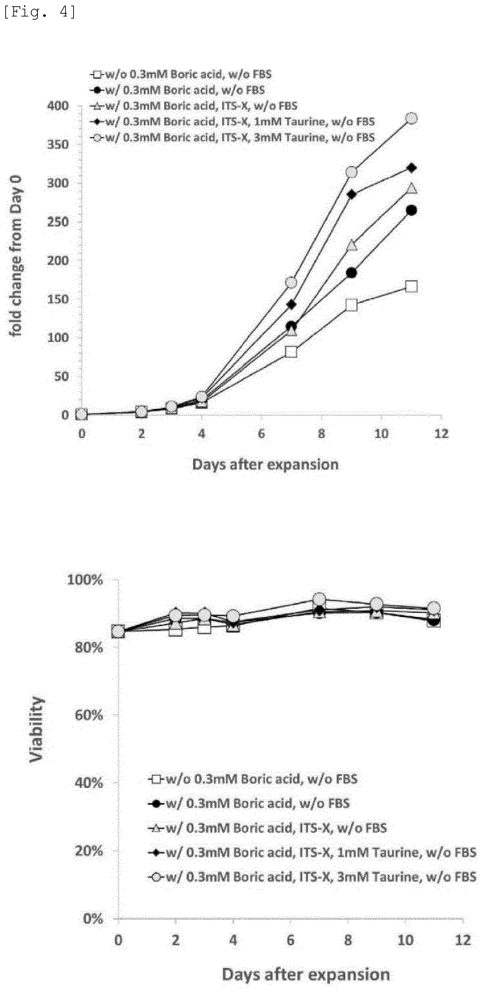

   Fig. 4 is a graph showing the results of a cell proliferation test of iPS cell-derived CAR-T cells that verify the effect of adding ITS-X alone and ITS-X in combination with taurine to a serum-free medium containing 0.3 mM boric acid. The upper row shows the cell proliferation fold from day 0 of expansion culture, and the lower row shows the measurement results of the cell viability. w/o; without, w/; with
[Fig. 5]

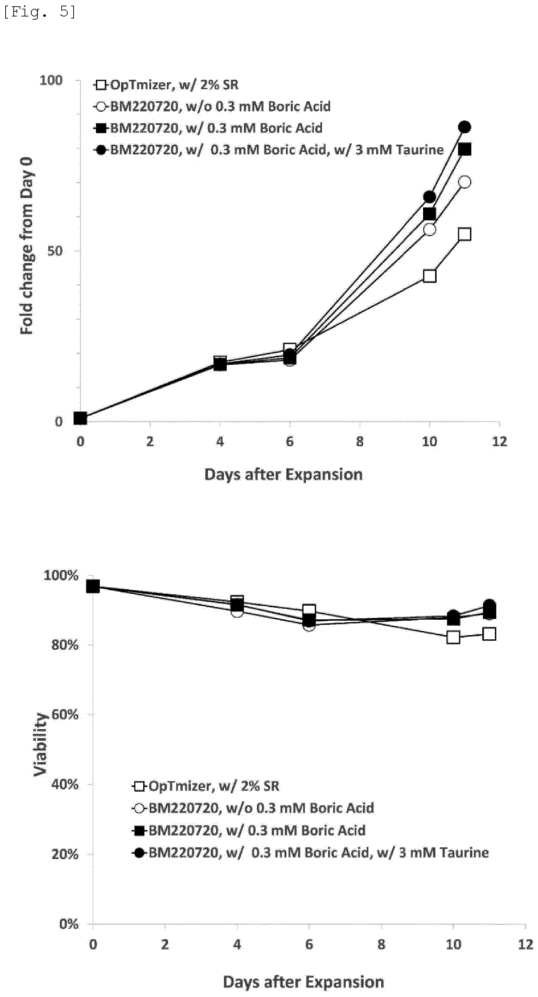

   Fig. 5 is a graph showing the results of a cell proliferation test of human-derived primary T cell-derived CAR-T cells that verify the effect of adding taurine alone to a serum-free medium containing 0.3 mM boric acid. The upper row shows the cell proliferation fold from day 0 of expansion culture, and the lower row shows the measurement results of the cell viability. w/o; without, w/; with

### (Detailed Description of the Invention)

The present invention is described in the following. The terms used in the present specification have the meanings generally used in the pertinent field unless otherwise specified.

### (1) Cell culture method (hereinafter also to be referred to as "the culture method of the present invention"), cell proliferation method (hereinafter also to be referred to as "the proliferation method of the present invention")

The culture method or proliferation method of the present invention contains a step of culturing cells in a serum-free medium or low-serum medium containing boric acid or a salt thereof.

### 1-1. Cell

The type of cell to be the target of culture in the culture method and proliferation method of the present invention is not particularly limited. Examples of the cell type include reproductive cells such as sperm and eggs, somatic cells that compose living organisms, stem cells (pluripotent stem cells, etc.) and cells induced to differentiate from stem cells, progenitor cells, cancer cells isolated from living organisms, cells (cell lines) isolated from living organisms that have acquired immortalization ability and are stably maintained outside the body, cells isolated from living organisms that have been artificially modified genetically, and cells isolated from living organisms and having artificially-exchanged nucleus. Examples of the somatic cells that compose living organisms and the cells induced to differentiate from stem cells include, but are not limited to, immunocompetent cells (natural killer (NK) cells, macrophages, monocytes, mast cells, dendritic cells, Langerhans cells, neutrophils, eosinophils, basophils, B cells, T cells, etc.), fibroblasts, bone marrow cells, red blood cells, platelets, bone cells, pericytes, keratinocytes, adipocytes, mesenchymal cells, epithelial cells, epidermal cells, endothelial cells, vascular endothelial cells, hepatic parenchymal cells, chondrocytes, cumulus cells, nervous system cells, glial cells, neurons, oligodendrocytes, microglia, astrocytes, cardiac cells, esophageal cells, muscle cells (e.g., smooth muscle cells or skeletal muscle cells), pancreatic beta cells, melanocytes, hematopoietic progenitor cells (e.g., CD34-positive cells derived from umbilical cord blood), and mononuclear cells. Somatic cells include cells obtained from any tissue, such as skin, kidney, spleen, adrenal gland, liver, lung, ovary, pancreas, uterus, stomach, colon, small intestine, large intestine, bladder, prostate, testes, thymus, muscle, connective tissue, bone, cartilage, vascular tissue, blood (including umbilical cord blood), bone marrow, heart, eye, brain, or neural tissue.

Stem cell is a cell that has the ability to replicate itself and to differentiate into other multiple lineages of cells. Examples thereof include, but are not limited to, embryonic tumor cells, pluripotent stem cells, neural stem cells, hematopoietic stem cells, mesenchymal stem cells, liver stem cells, pancreatic stem cells, muscle stem cells, germline stem cells, intestinal stem cells, cancer stem cells, and hair follicle stem cells.

"Pluripotent stem cells" refer to cells that have the ability to self-replicate and differentiate/proliferate, and have the ability to differentiate into all tissues and cells that constitute a living organism. Examples of pluripotent stem cells include embryonic stem cells (ES cells), embryonic germ cells (EG cells), induced pluripotent stem cells (iPS cells), and pluripotent stem cells induced and selected by stress or cell stimulation. Stem cells established by culturing early embryos produced by nuclear transplantation of the nucleus of a somatic cell are also preferred as pluripotent stem cells (Nature, 385, 810 (1997); Science, 280, 1256 (1998); Nature Biotechnology, 17, 456 (1999); Nature, 394, 369 (1998); Nature Genetics, 22, 127 (1999); Proc. Natl. Acad. Sci. USA, 96, 14984 (1999); Nature Genetics, 24, 109 (2000)). In the present invention, iPS cells are preferred as pluripotent stem cells. iPS cells can be identified using undifferentiated markers due to the undifferentiated nature of iPS cells as indicators. Examples of undifferentiated markers include alkaline phosphatase, Oct3/4, Sox2, Nanog, ERas, Esgl, etc. The methods for detecting these undifferentiated markers include methods for detecting mRNA (using primers and probes), immunological detection methods (using antibodies and labels), and the like.

A cell induced to differentiate from a stem cell (differentiation-induced cell) is any cell that has been subjected to a differentiation-inducing treatment that induces differentiation from a stem cell into a specific type of cell.

A cell line is a cell that has acquired the ability to proliferate indefinitely through artificial manipulation outside of a living organism. Examples thereof include, but are not limited to, CHO (Chinese hamster ovary cell line), HCT116, Huh7, HEK293 (human embryonic kidney cell), HeLa (human uterine cancer cell line), HepG2 (human hepatoma cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (trade name), Vero and the like.

In one preferred embodiment, the cell to be the target of culture is an immunocompetent cell. The immunocompetent cells refer to cells involved in various immune responses in vivo. Examples of immunocompetent cells include natural killer (NK) cells, macrophages, monocytes, mast cells, dendritic cells, Langerhans cells, neutrophils, eosinophils, basophils, B cells, and T cells, and are preferably selected from dendritic cells, B cells, T cells, and natural killer (NK) cells. More preferably, the immunocompetent cells are T cells.

T cells include CD4 positive CD8 negative T cells, CD4 negative CD8 positive T cells, αβ-T cells, γδ-T cells, regulatory T cells, and NKT cells. T cells may be subsets such as naive T cells, effector T cells, memory T cells, or the like.

The immunocompetent cells may be cells isolated from humans (primary cells) or cells obtained by differentiation from stem cells such as pluripotent stem cells (e.g., iPS cells, ES cells), hematopoietic stem cells, and mesenchymal stem cells, and are preferably primary cells and cells obtained by differentiation from pluripotent stem cells (particularly iPS cells). As the primary cells, primary T cells derived from human are preferred. Differentiation induction from stem cells to immunocompetent cells can be performed by a method known in the art, depending on the type of stem cells used and the type of immunocompetent cells of interest. In one embodiment, differentiation induction from iPS cells to T cells and differentiation induction from iPS cells to NK cells can be performed by the methods described in the Examples.

In addition, the cells to be the target may be either autologous cells or allogeneic cells. In the present invention, the "autologous cells" refers to cells obtained from a subject receiving a cell population (described below) produced by the culture method of the present invention or the method of the present invention, or cells derived from the obtained cells, and "allogeneic cells" refers to cells that are not the above-mentioned "autologous cells".

In the present invention, the cell to be the target of culture may be a cell that has been artificially modified genetically. Examples of such cells include immunocompetent cells into which a CAR gene has been introduced (T cells into which a CAR gene has been introduced and natural killer cells into which a CAR gene has been introduced), T cells into which an exogenous T cell receptor (TCR) has been introduced, and immunocompetent cells into which genes for expressing cytokines and/or chemokines have been introduced.

CAR is a structure including, from the N-terminal side to the C-terminal side of a protein, an extracellular domain specific to a target, a transmembrane domain, and an intracellular signal domain for the effector function of immune cells, and the CAR gene is a gene encoding this receptor. The extracellular domain contains an antigen recognition site that exhibits specific binding property to a target. The transmembrane domain is present between the extracellular domain and the intracellular signal domain. The intracellular signal domain transmits a signal necessary for immune cells to exert the effector functions thereof. That is, an intracellular signal domain capable of transmitting a signal necessary for activating immune cells when the extracellular domain binds to a target antigen is used. There have been several reports of experiments, clinical studies, and the like using CARs (e.g., Rossig C, et al. Mol Ther 10:5-18, 2004; Dotti G, et al. Hum gene Ther 20:1229-1239, 2009; Ngo MC, et al. Hum Mol Genet 20 (R1):R93-99, 2011; Ahmed N, et al. Mol Ther 17:1779-1787, 2009; Pule MA, et al. Nat Med 14:1264-1270, 2008; Louis CU, et al. Blood 118:6050-6056, 2011; Kochenderfer JN, et al. Blood 116:4099-4102, 2010; Kochenderfer JN, et al. Blood 119 :2709-2720, 2012; Porter DL, et al. N Engl J Med 365:725-733, 2011; Kalos M, et al. Sci Transl Med 3:95ra73,2011; Brentjens RJ, et al. Blood 118:4817-4828, 2011; Brentjens RJ, et al. Sci Transl Med 5:177 ra38, 2013), and the CAR in the present invention can be constructed by referring to these reports.

Preferred one embodiment of cells to which the culture method and proliferation method of the present invention are applied includes T cells into which a CAR gene has been introduced (CAR-T cells) and NK cells into which a CAR gene has been introduced (CAR-NK cells), more preferably CAR-T cells and CAR-NK cells derived from pluripotent stem cells, further preferably CAR-T cells derived from iPS cells and CAR-NK cells derived from iPS cells, and further more preferably CAR-T cells derived from iPS cells. CAR-T cells and CAR-NK cells can be obtained by introducing a CAR gene into T cells and NK cells, or their precursor cells such as pluripotent stem cells, respectively. For example, the CAR-T cells may be cells obtained by introducing a CAR gene into cells obtained by differentiating cells such as iPS cells, ES cells, hematopoietic stem cells, and mesenchymal stem cells into T cells, or cells obtained by differentiating iPS cells, into which a CAR gene has been introduced, into T cells. Preferably, the CAR-T cells are derived from iPS cells and obtained by differentiating iPS cells, into which a CAR gene has been introduced, into T cells, or the CAR-T cells are derived from iPS cells and obtained by introducing a CAR gene into T cells obtained by differentiating iPS cells, and these are sometimes collectively referred to as "iPS cell-derived CAR-T cells". More preferably, the CAR-T cells are derived from iPS cells and obtained by introducing a CAR gene into T cells obtained by differentiating iPS cells. For example, in the case of CAR-NK cells, the CAR-NK cells may be cells obtained by introducing a CAR gene into cells obtained by differentiating cells such as iPS cells, ES cells, hematopoietic stem cells, and mesenchymal stem cells into NK cells, or cells obtained by differentiating iPS cells, into which a CAR gene has been introduced, into NK cells. Preferably, the CAR-NK cells are derived from iPS cells and obtained by differentiating iPS cells, into which a CAR gene has been introduced, into NK cells, or the CAR-NK cells are derived from iPS cells and obtained by introducing a CAR gene into NK cells obtained by differentiating iPS cells, and these are sometimes collectively referred to as "iPS cell-derived CAR-NK cells". More preferably, the CAR-NK cells are derived from iPS cells and obtained by introducing a CAR gene into NK cells obtained by differentiating iPS cells.

The induction of differentiation from iPS cells (into which a CAR gene may be introduced, when desired) into T cells, or the induction of differentiation from iPS cells (into which a CAR gene may be introduced, when desired) into NK cells, can be performed using a known method, specifically, by the method described in the Examples.

The CAR gene is generally introduced into cells using a CAR expression vector. The CAR expression vector means a nucleic acid molecule capable of transporting a nucleic acid molecule encoding a CAR gene into cells. It may be DNA or RNA and is not particularly limited as to its form or origin, and various types of vectors may be used. The vector may be a viral vector or a non-viral vector. Examples of viral vectors include retroviral vectors, lentiviral vectors, adenoviral vectors, adeno-associated viral vectors, herpes viral vectors, Sendai viral vectors, and vaccinia viral vectors. Among these, in retroviral vectors, lentiviral vectors, and adeno-associated viral vectors, the target gene incorporated into the vector is incorporated into the host chromosome, and stable and long-term expression can be expected. Each viral vector can be produced according to a conventional method or using a commercially available dedicated kit. Examples of non-viral vectors include plasmid vectors, liposome vectors, positively charged liposome vectors (Felgner, P.L., Gadek, T.R., Holm, M. et al., Proc. Natl. Acad. Sci., 84:7413-7417, 1987), YAC vectors, BAC vectors, and artificial chromosome vectors.

In one embodiment, the CAR gene can be introduced into cells by the method described in the Examples.

### 1-2. Medium

The medium to be used in the culture method and proliferation method of the present invention is a serum-free medium or low-serum medium containing boric acid or a salt thereof (hereinafter sometimes collectively referred to as "the medium of the present invention").

In the present specification, boric acid is not particularly limited as long as it is pharmaceutically acceptable, and examples thereof include orthoboric acid, metaboric acid, and tetraboric acid. Among these boric acids, orthoboric acid is preferably used. Salts of boric acid are not particularly limited as long as they are pharmaceutically acceptable, and examples thereof include alkali metal salts such as sodium salts and potassium salts; alkaline earth metal salts such as calcium salts and magnesium salts; aluminum salts; and a salt with an organic amine such as triethylamine, triethanolamine, morpholine, piperazine, and pyrrolidine. In addition, boric acid or a salt thereof may be in the form of a hydrate, such as borax. The concentration of boric acid or a salt thereof in the medium is not particularly limited as long as the desired effect is obtained. It is generally 0.1 to 0.9 mM, preferably 0.2 to 0.8 mM, further preferably 0.2 to 0.7 mM, particularly preferably 0.3 to 0.6 mM. In the present specification, the concentration of boric acid or a salt thereof is, unless otherwise specified, the concentration converted to boric acid. When the concentration is too high, it affects the cell viability, and when it is too low, the effect of promoting cell proliferation is weak.

In the present specification, the "serum-free medium" means a medium that is substantially free of, preferably free of, unconditioned or unpurified serum, and a medium that is contaminated with purified blood-derived components or animal tissue-derived components (e.g., growth factors) is considered to be serum-free medium. As a medium, a medium used for culturing animal cells (for convenience, hereinafter also referred to as a basal medium) or a medium for perfusion culture is generally used. Here, "substantially free of" means that it does not contain at all, or even if it contains, it is below the detection limit.

Examples of basal media include, but are not limited to, BME medium, BGJb medium, CMRL 1066 medium, Glasgow MEM medium, Improved MEM ZincOption medium, IMDM medium, Medium 199 medium, Eagle MEM medium, αMEM medium, DMEM medium, Ham's medium, RPMI 1640 medium, Fischer's medium, F12 medium, and mixed media thereof (e.g., Advanced DMEM/F12 medium, etc.).

Perfusion culture is a culture method in which a culture system containing cells is continuously supplied with the medium while an equal amount of cell-free culture supernatant is continuously removed from the culture system to maintain the culture system in a steady state. Because perfusion culture requires a higher cell density than general culture methods, the medium for perfusion culture generally has a higher concentration of nutrient components than basal medium.

These media can be purchased from Invitrogen, SIGMA, FUJIFILM Wako Pure Chemical Corporation, Sumitomo Pharma, etc., and media with the same name or the same product name have the same composition regardless of manufacturer.

In the present specification, the "low-serum medium" refers to a medium in which serum has been added to a basal medium and characterized in that the serum concentration is reduced compared to serum-containing media used generally (serum concentration: 5-20% (serum concentration is expressed in v/v% in the present specification). The serum concentration of the low-serum medium may be less than 5%, less than 2%, less than 1%, less than 0.1%, less than 0.01%, or less than 0.001%, but is generally 0.1% or more, or 1% or more. Specifically, the aforementioned serum concentration is preferably 0.1% or more and less than 5%, more preferably 1% or more and less than 4%.

The medium used in this step may also contain a serum replacement. Examples of the serum replacement include albumin (e.g., lipid-rich albumin), transferrin, fatty acids, collagen precursors, insulin, trace elements (e.g., zinc, selenium, etc.), ITS supplement (mixture of insulin, transferrin, and selenite), B-27 supplement, N2 supplement, knockout serum replacement, 2-mercaptoethanol or 3'-thiolglycerol, or equivalents thereof. These serum replacements are also commercially available. Knockout serum replacement can be purchased from Invitrogen. Other serum replacements can be purchased from Invitrogen, SIGMA, FUJIFILM Wako Pure Chemical Corporation, Sumitomo Pharma, and the like, and the composition of reagents or additives with the same name or trade name is equivalent regardless of the manufacturer.

The medium of the present invention may further contain, in addition to the basal medium, components that are favorable for cell proliferation. Examples of such components include sugars such as glucose, fructose, sucrose, and maltose; amino acids or amino acid derivatives such as glycine, serine, alanine, valine, leucine, isoleucine, methionine, phenylalanine, tyrosine, tryptophan, proline, threonine, cysteine, asparagine, glutamine, aspartic acid, glutamic acid, arginine, lysine, histidine, ornithine, theanine, citrulline, taurine, betaine, carnitine, creatine, and pantothenic acid; proteins such as albumin and transferrin; peptides such as glycylglycylglycine and soybean peptide; serum; choline, vitamins such as vitamin B group (thiamine, riboflavin, pyridoxine, cyanocobalamin, biotin, folic acid, pantothenic acid, nicotinamide, etc.), vitamin C (ascorbic acid), and vitamin C derivatives; fatty acids such as oleic acid, arachidonic acid, and linoleic acid; lipids such as cholesterol; nucleosides such as adenosine, thymidine, guanosine, cytidine, uridine, and inosine; inosinic acid; hypoxanthine; inorganic salts such as sodium chloride, potassium chloride, calcium chloride, magnesium sulfate, and sodium dihydrogen phosphate; trace elements such as zinc, copper, selenium, vanadium, manganese, nickel, silicon, tin, molybdenum, cadmium, chromium, silver, aluminum, barium, cobalt, germanium, iodine, bromine, fluorine, rubidium, and zirconium; buffering agents such as N,N-bis(2-hydroxyethyl)-2-aminoethanesulfonic acid (BES), 4-(2-hydroxyethyl)-1-piperazineethanesulfonic acid (HEPES), and N-[tris(hydroxymethyl)methyl]glycine (Tricine); antibiotics such as amphotericin B, kanamycin, gentamicin, and streptomycin; cell adhesion factors and extracellular matrix components such as Type I collagen, Type II collagen, fibronectin, laminin, poly-L-lysine, and poly-D-lysine; cytokines and growth factors such as interleukins, fibroblast growth factors (FGF), hepatocyte growth factors (HGF), transforming growth factors (TGF)-α, transforming growth factors (TGF)-β, vascular endothelial growth factors (VEGF), and activin A; and hormones such as dexamethasone, hydrocortisone, estradiol, progesterone, glucagon, and insulin. Appropriate components can be selected and used at appropriate concentrations according to the type of cells to be cultured. In particular, taurine, which is one type of amino acid derivative, can be added to improve viability and proliferation of the cell, and can be used at a final concentration of generally 0.5 to 10 mM, preferably 2 to 5 mM. The growth factor may be from an animal species other than human, but is preferably from human origin (which may be recombinant).

The medium may further contain an additive factor to support cell survival and proliferation. The additive factor may be a type 1 cytokine family member, a type 2 cytokine family member, a TNF superfamily cytokine, an IL-1 family cytokine, or other cytokines (TNF-β, etc.), specifically IL-1 to IL-41, and the like, preferably IL-1, IL-2, IL-7, IL-15, IL-18, IL-21, and the like. The additive factor may be prepared according to a conventional method, or a commercially available product may be used. The additive factor may be from an animal species other than human, but is preferably from human origin (which may be recombinant).

In the present specification, "xeno-free" refers to conditions under which components derived from organisms other than the organism of the cells to be cultured are excluded.

### 1-3. Cell culture

The present invention also provides a method for culturing cells, characterized in that the cells are cultured in the medium of the present invention (described above) (hereinafter sometimes referred to as "the culture method of the present invention").

The incubator to be used for culturing the cells is not particularly limited as long as it is capable of culturing cells, and examples of the incubator include flasks, tissue culture flasks, dishes, Petri dishes, tissue culture dishes, multi-dishes, microplates, microwell plates, multi-plates, multi-well plates, microslides, chamber slides, petri dishes, tubes, trays, culture bags, roller bottles, and bioreactors. An appropriate incubator is selected according to the target cells.

The incubator may be either cell-adhesive or cell-non-adhesive, and is appropriately selected according to the purpose. A cell-adhesive incubator may be coated with any cell-supporting substrate, such as an extracellular matrix (ECM), in order to improve adhesion of the surface of the incubator to cells. The cell-supporting substrate may be any substance intended for cell adhesion.

Other culture conditions may be appropriately set. For example, the culture temperature is not particularly limited and may be about 30 to 40°C, preferably about 37°C. The CO₂ concentration may be about 1 to 10%, preferably about 2 to 5%. The oxygen partial pressure may be 1 to 21%.

The frequency of medium exchange and the culture period in cell culture are generally determined by comprehensively considering various conditions such as cell density, culture method (adhesive culture/suspension culture), type of cell to be cultured, medium composition, culture conditions (temperature, gas concentration), amount of medium to be exchanged (total amount/partial amount), cost of medium, and lifestyle of the operator. Medium exchange is generally performed once every 2 to 3 days, once a day, or multiple times a day (e.g., twice a day), and the medium exchange can be performed at such frequencies in the culture method and proliferation method of the present invention. Furthermore, medium exchange can be performed continuously by perfusion culture. The culture period is generally about 4 days to 4 weeks, preferably about 4 days to 3 weeks, more preferably about 1 to 2 weeks.

In one embodiment of the present invention, the cells to be cultured in a serum-free medium or low-serum medium containing boric acid or a salt thereof (i.e., the medium of the present invention) are immunocompetent cells, preferably selected from dendritic cells, B cells, T cells, and natural killer (NK) cells, more preferably T cells and NK cells, and further preferably T cells. In another embodiment of the present invention, the cells to be cultured in the medium of the present invention are primary T cells and T cells derived from pluripotent stem cells (particularly iPS cells), more preferably primary T cell-derived and pluripotent stem cell (particularly iPS cell)-derived CAR-T cells, further preferably pluripotent stem cell (particularly iPS cell)-derived CAR-T cells. In still another embodiment of the present invention, the cells to be cultured in the medium of the present invention are primary NK cells and pluripotent stem cell (particularly iPS cell)-derived NK cells, more preferably primary NK cell-derived and pluripotent stem cell (particularly iPS cell)-derived CAR-NK cells, further preferably pluripotent stem cell (particularly iPS cell)-derived CAR-NK cells.

In one embodiment of the present invention, culture includes an activation step and an expansion step. When cryopreserved cells are used, a recovery step may be included. In the present invention, when the culture target is a cryopreserved cell, the culture of the cell is a concept including any one, two, or all three of recovery culture consisting of a recovery step, activation culture consisting of an activation step, and expansion culture consisting of an expansion step. Preferably, the target is expansion culture. Another embodiment of the present invention is a culture of CAR-T cells and CAR-NK cells, preferably pluripotent stem cell (particularly iPS cell)-derived CAR-T cells and pluripotent stem cell (particularly iPS cell)-derived CAR-NK cells, which includes an activation step and an expansion step, and includes, where necessary, a recovery step (in the case of using cryopreserved cells, etc.).

### (Recovery step/recovery culture)

This step is performed after thawing the cryopreserved cells. Since prolonged exposure of cells to a cryoprotectant can cause damage, the cryoprotectant is promptly removed from the cells after thawing. Then, cell damage and cell dysfunction caused by freeze-thawing are restored. This step/culture method varies depending on the target cells, the cryoprotectant used, the thawing method, and the like. Most cells generally recover normally by culturing them in a medium that does not contain a cryoprotectant for several days, preferably 1 to 5 days, more preferably about 3 days.

### (Activation step/activation culture)

The method of this step is not particularly limited as long as the desired effect on the cells can be obtained, and for example, it can be performed by contacting with a stimulating substance. Generally, the cells are cultured in the presence of a stimulating substance, specifically in a medium containing the stimulating substance, for several days, preferably 1 to 5 days, more preferably about 3 days. The stimulating substance is a signal molecule that controls the activity of cells by a method such as autocrine, paracrine, or endocrine, and may be a substance that can be secreted from all cells contained in the culture system, or may be a substance that is added from the outside. Specifically, the step is carried out by contact with a substrate to which a ligand is bound, or by culturing in a medium containing a ligand, or the like.

For example, the ligand used in the activation culture of CAR-T cells, preferably pluripotent stem cell (particularly iPS cell)-derived CAR-T cells, is not particularly limited as long as it is a molecule that interacts with a surface molecule of CAR-T cells and promotes the activation thereof. Examples include CD3, which couples with TCR and is responsible for signal transduction via TCR, and molecules that specifically bind to surface molecules known as costimulatory factors for T cell activation, such as CD28, ICOS, CD137, OX40, CD27, GITR, BAFFR, TACI, BMCA, and CD40L, and have the function of transmitting an activation signal into T cells. Such molecules may be physiological ligands (or receptors) for the above-mentioned T cell surface molecules, or non-physiological ligands (or receptors) having agonistic activity. A preferred example of a non-physiological ligand is an agonist antibody.

More preferably, the T cell activation ligand to be used in the present invention is an antibody against CD3. The antibody against CD3 may be a complete antibody or a fragment thereof (e.g., Fab, F(ab')₂, Fab', scFv, Fv, reduced antibody (rIgG), dsFv, sFv, diabody, triabody, etc.) as long as it has the ability to specifically bind to CD3 expressed in target T cells that induce activation and to stimulate the surface molecules of these T cells to transmit a signal into the T cells.

For example, the molecule used in the activation culture of CAR-NK cells, preferably pluripotent stem cell (particularly iPS cell)-derived CAR-NK cells, is not particularly limited as long as it is a molecule that interacts with a surface molecule of CAR-NK cells and promotes the activation thereof. For example, it may be a physiological ligand (or receptor) for the above-mentioned NK cell surface molecule, or a non-physiological ligand (or receptor) having agonistic activity. It may also be a cytokine. A preferred example of the non-physiological ligand is an antibody against CD3. The antibody may be a complete antibody or a fragment thereof (e.g., Fab, F(ab')₂, Fab', scFv, Fv, reduced antibody (rIgG), dsFv, sFv, diabody, triabody, etc.). Examples of cytokines include IFN-α/β, IFN-y, IL-2, IL-4, IL-7, IL-12, IL-15, IL-18, and IL-21.

### (Expansion step/expansion culture)

In this step, the cells activated as described above are proliferated. The method of expansion culture is not particularly limited as long as the desired effect on the activated cells is obtained, and a person skilled in the art can appropriately adjust the method while monitoring the number of cells, and the like. When the purpose is to increase the number of cells, the cells can be cultured in a medium containing the above-mentioned additives and the like for, for example, 2 days or more, preferably 3 days or more, more preferably 4 days or more, further preferably 5 days or more, and further more preferably 6 days or more. The cells can be cultured while monitoring the number of cells and the like and appropriately adjusting for, for example, 7 days or more, 9 days or more, or 11 days or more. In addition, the number of cells can be exponentially increased by continuing the culture by alternately repeating activation culture and expansion culture. The upper limit of the culture period is not particularly limited, and is, for example, 28 days or less, preferably 21 days or less. For example, in the case of activated CAR-T cells, preferably activated pluripotent stem cell (particularly iPS cell)-derived CAR-T cells, the cells can be expansion cultured in a medium containing cytokines such as IL-7, IL-15, and IL-2 for, for example, 2 days or more, preferably 3 days or more, more preferably 4 days or more, further preferably 5 days or more, and further more preferably 6 days or more. When the target cells are primary T cells (particularly CAR-T cells) or NK cells (preferably pluripotent stem cell (particularly iPS cell-derived)-derived NK cells (particularly CAR-NK cells)) and the purpose is to increase the number of cells, the cells can be expansion cultured in a medium containing cytokines such as IL-7, IL-15, IL-21, and IL-2 for, for example, 2 days or more, preferably 4 days or more, more preferably 6 days or more, further preferably 8 days or more, further more preferably 10 days or more, and even further more preferably 11 days or more.

### (2) A method for producing a cell population (hereinafter also referred to as the "production method of the present invention") and a medicament containing the cell population (hereinafter to be also referred to as the "medicament of the present invention")

The production method of the present invention is a method for producing a cell population in which the number of desired cells is expanded, and the method includes a step of culturing a cell population containing the desired cells in a serum-free medium or low-serum medium containing boric acid or a salt thereof.

The "serum-free medium or low-serum medium containing boric acid or a salt thereof" is the same as that described in the culture method of the present invention and the proliferation method of the present invention in the above-mentioned (1).

The desired cell is a cell the proliferation of which is aimed, and includes those exemplified in the above-mentioned section "1-1. Cell" in (1). It is preferably an immunocompetent cell, more preferably T cell or NK cell, particularly preferably T cell into which a CAR gene has been introduced, i.e., CAR-T cell, and NK cell into which a CAR gene has been introduced, i.e., CAR-NK cell. The CAR-T cells and CAR-NK cells are preferably derived from pluripotent stem cells, preferably iPS cells, and CAR-T cells derived from pluripotent stem cells (particularly iPS cells) are even more preferred.

The origin of the "cell population containing the desired cells" is not particularly limited as long as the desired cells are contained, and the cell population may be naturally derived or artificially prepared. For example, when immunocompetent cells such as T cells are desired, the cell population may be exemplified by cell populations containing peripheral blood mononuclear cells (PBMCs), blood cells, hematopoietic stem cells, cord blood mononuclear cells, and the like, collected, isolated, purified, or induced from body fluids such as blood (peripheral blood, umbilical cord blood, etc.) and bone marrow fluid. These cells may be collected from a living body or obtained through ex vivo culture and, for example, a cell population obtained by the production method of the present invention, either as is or frozen, can be mentioned. In addition, the cell population is not limited to a cell population of single cells, and may be a slice of a tissue mass obtained by biopsy, with preference given to a cell population of single cells. In the case of a humoral cell population, it is not limited to a cell population derived from a single tissue, and may be a mixed system of cell populations derived from different tissues. The "cell population containing the desired cells" is preferably a cell population containing immunocompetent cells, more preferably a cell population containing T cells or NK cells, and particularly preferably a cell population containing T cells into which a CAR gene has been introduced, i.e., CAR-T cells, or a cell population containing NK cells into which a CAR gene has been introduced, i.e., CAR-NK cells. A cell population containing CAR-T cells derived from pluripotent stem cells, preferably iPS cells, or a cell population containing CAR-NK cells derived from pluripotent stem cells, preferably iPS cells is even more preferred.

"The number of desired cells is expanded" refers to an increase in the number of desired cells in a cell population compared to a control, such as the number of cells before expansion or obtained without practicing the present invention, and means an increase of at least 10%, 20%, 30%, 40%, 50%, 60%, 70%, 80%, 90%, 100%, 120%, 150%, 200%, 300%, 400%, 500%, 600%, 700%, 800%, 900%, or 1000% compared to the control. In a specific embodiment of the present invention, the number of desired cells in the cell population produced by the present invention is expanded to achieve an increase by at least 10%, 20%, 30%, 40%, 50%, 60%, 70%, 80%, 90%, 100%, 120%, 150%, 200%, 300%, 400%, 500%, 600%, 700%, 800%, 900%, or 1000%, compared to the number of cells in the control.

When the desired cells are CAR-T cells or CAR-NK cells, the cells increase their number, i.e., are expanded, through activation culture and expansion culture (and recovery culture, where necessary), as described above. After expansion culture, the cells are washed and collected. When cultured or proliferated using the culture method and proliferation method of the present invention, since the medium used is a serum-free medium or a low-serum medium, the washing operation is simpler and the time required for washing can be shortened compared to when cultured or proliferated using a serum-containing medium (serum concentration: 5 to 20%) used generally.

For example, when cells are washed and collected using (i) pipetting and centrifugation or (ii) a closed-system automated cell processing device, continuous automated processing using techniques such as (i) pipetting and centrifugation or (ii) closed-system centrifugation/spinning membrane filtration/hollow fiber membrane filtration needs to be repeated multiple times until the BSA contained in the serum reaches an amount acceptable for administration to human. When cultured or proliferated using the culture method and proliferation method of the present invention, multiple repetitions are not necessary (the amount of BSA derived from serum brought into the culture system is originally small). Alternatively, the desired cell population can be collected by fewer repetitions compared to when a serum-containing medium (serum concentration: 5 to 20%) is used.

In one preferred embodiment, a step of culturing the cells (after expansion culture) in the presence of a stimulating substance is performed before the collection operation. This step allows efficient expansion culture, and also has the advantage of increasing the viability of the cells. When desired, the CAR-T cell population or CAR-NK cell population after expansion culture may be subjected to a washing operation and a cell separation step using beads separation or the like. This embodiment makes it possible to increase the purity of a CAR-T cell population or CAR-NK cell population with higher effects. Also in this embodiment, when cultured or expanded using the culture method and proliferation method of the present invention, the washing operation can be simplified and/or the time required for washing can be shortened.

In this way, the number of desired cells is expanded in the cell population obtained by the production method of the present invention.

The "cell population in which the number of desired cells is expanded" produced by the production method of the present invention can produce a medicament as appropriate depending on the function of the cells. When the cells are CAR-T cells or CAR-NK cells, the cell population containing CAR-T cells or CAR-NK cells produced by the culture method and proliferation method of the present invention can be used to treat cancer, particularly cancer that expresses the target antigen of the CAR-expressing immune cells. The cancer may be a solid tumor or a blood tumor. Specific examples of cancer include, but are not limited to, various B-cell lymphomas (follicular malignant lymphoma, diffuse large B-cell malignant lymphoma, mantle cell lymphoma, MALT lymphoma, intravascular B-cell lymphoma, CD20-positive Hodgkin's lymphoma, etc.), myeloproliferative neoplasms, myelodysplastic/myeloproliferative neoplasms (CMML, JMML, CML, MDS/MPN-UC), myelodysplastic syndromes, acute myeloid leukemia, neuroblastoma, brain tumors, Ewing's sarcoma, osteosarcoma, retinoblastoma, small cell lung cancer, non-small cell lung cancer, melanoma, bone and soft tissue sarcoma, kidney cancer, pancreatic cancer, malignant mesothelioma, prostate cancer, breast cancer, uterine cancer, cervical cancer, ovarian cancer, and colorectal cancer. In a preferred embodiment, the cancer is a solid tumor. Examples of solid tumors include neuroblastoma, brain tumor, Ewing's sarcoma, osteosarcoma, retinoblastoma, small cell lung cancer, non-small cell lung cancer, melanoma, ovarian cancer, rhabdomyosarcoma, bone and soft tissue sarcoma, kidney cancer, pancreatic cancer, malignant mesothelioma, prostate cancer, breast cancer, uterine cancer, cervical cancer, ovarian cancer, and colorectal cancer.

The medicament of the present invention is administered in a therapeutically effective amount determined appropriately according to the age, weight, body surface area, symptoms, and the like of the subject. The subject in this disclosure is a mammal, particularly, generally a human, and preferably a cancer patient. The medicament of the present invention can be administered at, for example, 1x10⁴ cells to 1x10¹⁰ cells per administration. The administration route is not particularly limited, and it can be administered intratumorally, peritumorally, intraventricularly, intravenously, intraarterially, intraportally, intradermally, subcutaneously, intramuscularly, or intraperitoneally. The cell population of the present disclosure may be administered systemically or locally. Local administration includes direct injection into the target tissue, internal organ, or organ. The administration schedule is appropriately determined according to the age, weight, body surface area, symptoms, and the like of the subject, and may be a single administration or continuous or regular multiple administrations. The medicament of the present invention may be used for autotransplantation or allotransplantation. It may also be used in combination with other medicaments.

In particular, the medicament of the present invention may contain, in addition to the cell population to be administered to the subject, components such as dimethyl sulfoxide (DMSO) or serum albumin for the purpose of protecting the cells, antibiotics for the purpose of preventing bacterial contamination, and various components (vitamins, cytokines, growth factors, steroids, etc.) for the purpose of activating, proliferating, or inducing differentiation of cells. The composition can be prepared by a conventional method (for example, a method described in the Japanese Pharmacopoeia, etc.).

### (3) Medium additive (hereinafter also to be referred to as "the medium additive of the present invention") and medium composition containing the medium additive (hereinafter also to be referred to as "the medium composition of the present invention")

The medium additive of the present invention contains boric acid or a salt thereof. The medium additive of the present invention is used for addition to a serum-free medium or a low-serum medium. The "boric acid or a salt thereof" is the same as that described in the culture method of the present invention and the proliferation method of the present invention in the above-mentioned (1). The amount of boric acid or a salt thereof to be added to the medium additive is not particularly limited as long as the desired effect is obtained. It may be generally 0.01 to 100% by weight, preferably 0.1 to 100% by weight, more preferably 1 to 100% by weight, further preferably 5 to 100% by weight, particularly preferably 10 to 100% by weight.

The medium composition of the present invention is a composition containing medium components obtained by adding the medium additive of the present invention to a serum-free medium or a low-serum medium. As used herein, the "serum-free medium or low-serum medium" has the same meaning as the "serum-free medium or low-serum medium" described in the above-mentioned (1) in the culture method of the present invention and the proliferation method of the present invention, except that boric acid or a salt thereof has been removed. The medium additive of the present invention is added to a "serum-free medium or low-serum medium" such that the concentration of boric acid or a salt thereof in the medium composition is within the range of 0.1 to 0.9 mM, preferably 0.2 to 0.8 mM, further preferably 0.2 to 0.7 mM, particularly preferably 0.3 to 0.6 mM.

The present invention is described in detail in the following using Examples, but the present invention is not limited in any way. Unless otherwise specified, the reagents and materials to be used are commercially available or can be prepared according to known literatures and the like. Furthermore, those skilled in the art understand that any substance having a similar effect or action can be used alternatively.

### [Example]

### (List of Abbreviations)

BMP-4: bone morphogenetic protein-4
bFGF: basic fibroblast growth factor
VEGF: vascular endothelial growth factor
SCF: stem cell factor
TPO: thrombopoietin
FLT3L: Fms-related tyrosine kinase 3 ligand
Fc-DLL4: Recombinant Human DLL4 Fc Chimera Protein
DLL4: Delta-like protein 4
IL7: Interleukin-7
SDF1α: Stromal cell-derived factor 1α
αMEM: alpha Modified Eagle Minimum Essential medium
TCR: T-cell receptor

### Example 1

### 1. Preparation of iPS cell-derived CAR-T cell

The TCR gene was introduced into the iPS cell line QHJI01S04 provided by the Center for iPS Cell Research and Application (CiRA), Kyoto University, using a lentiviral vector. The iPS cells into which the TCR gene was introduced were differentiated into hematopoietic progenitor cells with reference to the known method described in Nature Communication 2021; 12: 430. Specifically, the iPS cells were cultured for 4 days in the presence of CHIR99021, SB431542, BMP-4, bFGF, VEGF to induce differentiation into mesoderm. Furthermore, the hematopoietic cytokines SCF, TPO, and FLT3L were used to differentiate into hematopoietic progenitor cells. The differentiation of the obtained hematopoietic progenitor cells into cytotoxic T lymphocytes (CTLs) was performed according to a known patent method (WO2017/221975) and literature information (Nature Communication 2021; 12: 430). Specifically, from the obtained hematopoietic progenitor cells, CD34-positive cells were purified using magnetic beads (Myltenyi Biotec), and cultured for 3 weeks in α-MEM medium containing SCF, TPO, FLT3L, IL7, SDF1α, and SB203580 on a plate on which Fc-DLL4 and RetroNectin (Recombinant Human Fibronectin Fragment, TakaraBio) were immobilized. A CAR gene that recognizes a specific antigen was introduced into the obtained TCR-positive CTL by using a retroviral vector. In the following, the cells were cryopreserved in liquid nitrogen and used as iPS cell-derived CAR-T cells.

### 2. Recovery culture of iPS cell-derived CAR-T cell

The iPS cell-derived CAR-T cells were suspended at 500,000 cells/mL in IMDM medium containing 15% Fetal Bovine Serum (FBS) to which the additives shown in the "recovery culture medium" column in Table 1 were added, seeded on G-Rex (registered trademark) 10M (Wilson Wolf), and cultured for 3 days under 5% CO₂/37°C.

**[Table 1]**

| additive to medium | manufacturer | final concentration | recovery culture medium | activation culture medium | expansion culture medium |
|---|---|---|---|---|---|
| L-Glutamine (200 mM) | Thermo Fisher | 2 mM | + | + | + |
| Streptomycin Sulfate | MEIJI | 100 µg/mL | + | + | + |
| ITS (100x) (Insulin-Transferrin-Selenium Supplements) | Gibco | 1x | + | + | |
| Ascorbic Acid 2-Phosphate | Sigma-Aldrich | 50 µg/mL | + | + | + |
| IL-7 | Peprotech | 10 ng/mL | + | + | + |
| IL-15 | Peprotech | 10 ng/mL | + | + | + |
| IL-2 | Peprotech | 100 ng/mL | + | + | + |
| IL-18 | MBL | 50 ng/mL | | + | |
| IL-21 | Peprotech | 20 ng/mL | | + | |
| Caspase Inhibitor Z-VAD-FMK | R&D | 10 µM | | + | |
| Human CD30 antibody | R&D | 300 ng/mL | | + | |
| Human Serum Albumin 12.5 g/50 mL | CSL Behring | 2 g/L | | | + |

| | | | | | |
|---|---|---|---|---|---|
| Additives added to each group are indicated with +. | | | | | |

### 3. Reagent, antibody

RetroNectin (trade name) was purchased from Takara Bio Inc. The anti-CD3 agonist antibody used was Anti-CD3 mAb GMP grade, Anti-CD3 monoclonal antibody (Clone: OKT3) purchased from Takara Bio Inc.

### 4. Immobilization of anti-CD3 agonist antibody and RetroNectin (trade name) on culture plate

Anti-CD3 agonist antibody (OKT3, final concentration 3 µg/mL) and RetroNectin (trade name) (final concentration 150 µg/mL) dissolved in PBS at the required concentration was added to a T225 flask and left standing overnight at 4°C. After washing with PBS, the cells were subjected to the test.

### 5. Activation culture of iPS cell-derived CAR-T cell

The iPS cell-derived CAR-T cells after recovery culture were suspended at 133,333 cells/mL in IMDM medium containing 15% FBS to which the additives shown in the "activation culture medium" column in Table 1 were added, seeded into a T225 flask to which an anti-CD3 agonist antibody (OKT3) and RetroNectin (trade name) were immobilized, and cultured for 3 days under 5% CO₂/37°C.

### 6. Expansion culture of iPS cell-derived CAR-T cells in two types of 1% FBS-containing media

A total of two types of expansion culture media (0 mM boric acid-added medium and 0.3 mM boric acid-added medium) were prepared: advanced DMEM/F12 medium (Thermo Fisher) containing 1% FBS, and further, additives shown in the "expansion culture medium" column in Table 1, and said medium further added with 0.3 mM boric acid (Sigma-Aldrich). After 3 days of activation culture, the cells were collected from the T225 flask and suspended in each of the two types of expansion culture media at 40,000 cells/mL, and cultured under 5% CO₂/37°C using G-Rex (registered trademark) 10M (Wilson Wolf). Then, on the fifth day of culture, some of the cells were recovered from G-Rex (registered trademark) 10M and the cells were counted, and the medium was replaced with the respective medium. On the sixth day of culture, some of the cells were recovered from G-Rex (registered trademark) 10M and the cells were counted. On the seventh day of culture, some of the cells were recovered from the plate, counted, and seeded onto a G-Rex (registered trademark) 24-well plate (Wilson Wolf) containing each medium at 1,375,000 cells/mL. Thereafter, on the 10th day of culture, some of the cells were collected from the G-Rex (registered trademark) 24-well plate, counted, and seeded at 50,000 cells/mL into G-Rex (registered trademark) 24-well plate (Wilson Wolf) containing respective media. On the 12th and 14th days of culture, some of the cells were similarly collected from the G-Rex (registered trademark) 24-well plate, counted, and the medium was replaced with each medium.

### Experimental Example 1

### Proliferation test to verify the effect of adding 0.3 mM boric acid to 1% FBS-containing medium

In the expansion culture of item 6 in Example 1, the cell number and viability of iPS cell-derived CAR-T cells were measured on days 3, 5, 6, 7, 12, and 14 of culture. The cell proliferation fold from day 0 of expansion culture is shown in Fig. 1. The proliferation of iPS cell-derived CAR-T cells was promoted by adding 0.3 mM boric acid to 1% FBS-containing medium (Fig. 1).

### Example 2

### 1. Recovery culture of iPS cell-derived CAR-T cell

The iPS cell-derived CAR-T cells were suspended at 500,000 cells/mL in IMDM medium containing 15% FBS to which the additives shown in the "recovery culture medium" column in Table 2 were added, seeded on G-Rex (registered trademark) 10M (Wilson Wolf), and cultured for 3 days under 5% CO₂/37°C.

**[Table 2]**

| additive to medium | manufacturer | final concentration | recovery culture medium | activation culture medium | expansion culture medium |
|---|---|---|---|---|---|
| L-Glutamine (200 mM) | Thermo Fisher | 2 mM | + | + | + |
| Streptomycin Sulfate | MEIJI | 100 µg/mL | + | + | + |
| ITS (100x) (Insulin-Transferrin-Selenium Supplements) | Gibco | 1x | + | + | |
| Ascorbic Acid 2-Phosphate | Sigma-Aldrich | 50 µg/mL | + | + | + |
| IL-7 | Peprotech | 10 ng/mL | + | + | + |
| IL-15 | Peprotech | 10 ng/mL | + | + | + |
| IL-2 | Peprotech | 100 ng/mL | + | + | + |
| IL-18 | MBL | 50 ng/mL | | + | |
| IL-21 | Peprotech | 20 ng/mL | | + | |
| Caspase Inhibitor Z-VAD-FMK | R&D | 10 µM | | + | |
| Human CD30 antibody | R&D | 300 ng/mL | | + | |
| Human Serum Albumin 12.5 g/50 mL | CSL Behring | 2 g/L | | | + |

| | | | | | |
|---|---|---|---|---|---|
| Additives added to each group are indicated with +. | | | | | |

### 2. Reagent, antibody

RetroNectin (trade name) was purchased from Takara Bio Inc. The anti-CD3 agonist antibody used was Anti-CD3 mAb GMP grade, Anti-CD3 monoclonal antibody (Clone: OKT3) purchased from Takara Bio Inc.

### 3. Immobilization of anti-CD3 agonist antibody and RetroNectin (trade name) on culture plate

Anti-CD3 agonist antibody (OKT3, final concentration 3 µg/mL) and RetroNectin (trade name) (final concentration 150 µg/mL) dissolved in PBS at the required concentration was added to a T225 flask and left standing overnight at 4°C. After washing with PBS, the cells were subjected to the test.

### 4. Activation culture of iPS cell-derived CAR-T cell

The iPS cell-derived CAR-T cells after recovery culture were suspended at 133,333 cells/mL in IMDM medium containing 15% FBS to which the additives shown in the "activation culture medium" column in Table 2 were added, seeded into a T225 flask to which an anti-CD3 agonist antibody (OKT3) and RetroNectin (trade name) were immobilized, and cultured for 3 days under 5% CO₂/37°C.

### 5. Expansion culture of iPS cell-derived CAR-T cells in two types of serum-free media

A total of two types of expansion culture media were prepared: an expansion culture medium (without addition of boric acid) which was Advanced DMEM/F12 medium containing additives shown in the "expansion culture medium" column in Table 2, and said medium further added with 0.3 mM boric acid. After 3 days of activation culture, the cells were collected from the T225 flask and suspended in each of the two types of expansion culture media at 40,000 cells/mL, and cultured under 5% CO₂/37°C using G-Rex (registered trademark) 10M (Wilson Wolf). Then, on the fifth day of culture, some of the cells were collected from G-Rex (registered trademark) 10M and counted, and the medium was replaced with each medium. On the 7th and 10th days of culture, the cells were counted by a similar method, seeded in a G-Rex (registered trademark) 24-well plate at 50,000 cells/mL, and cultured in each medium. On the 12th and 14th days of culture, some of the cells were similarly collected from the plate, counted, and the medium was replaced with each medium.

### Experimental Example 2

### Proliferation test to verify the effect of adding boric acid to serum-free expansion culture medium

In order to verify the influence of addition of boric acid to a serum-free expansion culture medium, in the expansion culture of item 5 in Example 2, the cell number and viability of iPS cell-derived CAR-T cells were measured on days 3, 5, 6, 7, 10, 12, and 14 of culture. The cell proliferation fold from day 0 of expansion culture is shown in Fig. 2. The proliferation of iPS cell-derived CAR-T cells was promoted by adding 0.3 mM boric acid to serum-free medium (Fig. 2).

### Example 3

### 1. Recovery culture of iPS cell-derived CAR-T cell

The iPS cell-derived CAR-T cells were suspended at 500,000 cells/mL in IMDM medium containing 15% FBS to which the additives shown in the "recovery culture medium" column in Table 3 were added, seeded on G-Rex (registered trademark) 10M (Wilson Wolf), and cultured for 3 days under 5% CO₂/37°C.

**[Table 3]**

| additive to medium | manufacturer | final concentration | recovery culture medium | activation culture medium | expansion culture medium |
|---|---|---|---|---|---|
| L-Glutamine (200 mM) | Thermo Fisher | 2 mM | + | + | + |
| Streptomycin Sulfate | MEIJI | 100 µg/mL | + | + | + |
| ITS (100x) (Insulin-Transferrin-Selenium Supplements) | Gibco | 1x | + | + | |
| Ascorbic Acid 2-Phosphate | Sigma-Aldrich | 50 µg/mL | + | + | + |
| IL-7 | Peprotech | 10 ng/mL | + | + | + |
| IL-15 | Peprotech | 10 ng/mL | + | + | + |
| IL-2 | Peprotech | 100 ng/mL | + | + | + |
| IL-18 | MBL | 50 ng/mL | | + | |
| IL-21 | Peprotech | 20 ng/mL | | + | |
| Caspase Inhibitor Z-VAD-FMK | R&D | 10 µM | | + | |
| Human CD30 antibody | R&D | 300 ng/mL | | + | |
| Human Serum Albumin 12.5 g/50 mL | CSL Behring | 2 g/L | | | + |

| | | | | | |
|---|---|---|---|---|---|
| Additives added to each group are indicated with +. | | | | | |

### 2. Reagent, antibody

RetroNectin (trade name) was purchased from Takara Bio Inc. The anti-CD3 agonist antibody used was Anti-CD3 mAb GMP grade, Anti-CD3 monoclonal antibody (Clone: OKT3) purchased from Takara Bio Inc.

### 3. Immobilization of anti-CD3 agonist antibody and RetroNectin (trade name) on culture plate

Anti-CD3 agonist antibody (OKT3, final concentration 3 µg/mL) and RetroNectin (trade name) (final concentration 150 µg/mL) dissolved in PBS at the required concentration was added to a T225 flask and left standing overnight at 4°C. After washing with PBS, the cells were subjected to the test.

### 4. Activation culture of iPS cell-derived CAR-T cell

The iPS cell-derived CAR-T cells after recovery culture were suspended at 133,333 cells/mL in IMDM medium containing 15% FBS to which the additives shown in the "activation culture medium" column in Table 3 were added, seeded into a T225 flask to which an anti-CD3 agonist antibody (OKT3) and RetroNectin (trade name) were immobilized, and cultured for 3 days under 5% CO₂/37°C.

### 5. Expansion culture of iPS cell-derived CAR-T cells in five types of serum-free media

A total of five types of expansion culture media were prepared: Advanced DMEM/F12 medium containing additives shown in the "expansion culture medium" column in Table 3, and further each, boric acid at 0.2 mM, 0.3 mM, 0.4 mM, 0.6 mM, or 0.9 mM. After 3 days of activation culture, the cells were collected from the T225 flask and suspended in each of the five types of expansion culture media at 50,000 cells/mL, and cultured under 5% CO₂/37°C using G-Rex (registered trademark) 24-well plate (Wilson Wolf). Then, on the fifth day of culture, some of the cells were recovered from G-Rex (registered trademark) 24-well plate and the cells were counted, and the medium was replaced with the respective medium. On the 7th day of culture, the cells were counted by a similar method, seeded in a fresh G-Rex (registered trademark) 24-well plate at 50,000 cells/mL, and cultured in each medium. On the 10th and 12th days of culture, some of the cells were similarly collected from the plate, counted, and the medium was replaced with each medium.

### Experimental Example 3

### Proliferation test of iPS cell-derived CAR-T cells when different concentrations of boric acid were added to serum-free expansion culture medium

To verify the optimal concentration of boric acid to be added to serum-free expansion culture medium, in the expansion culture in item 5 of Example 3, the number of iPS cell-derived CAR-T cells was counted over time. The cell proliferation fold from day 0 of expansion culture is shown in Fig. 3. The proliferation fold was maximized when the final concentration of boric acid was within the range of 0.3 mM to 0.6 mM. It was also confirmed that the cell viability was not influenced within the boric acid concentration range of 0.2 mM to 0.9 mM (Fig. 3).

### Example 4

### 1. Recovery culture of iPS cell-derived CAR-T cell

The iPS cell-derived CAR-T cells were suspended at 500,000 cells/mL in IMDM medium containing 15% FBS to which the additives shown in the "recovery culture medium" column in Table 4 were added, seeded on G-Rex (registered trademark) 10M (Wilson Wolf), and cultured for 3 days under 5% CO₂/37°C.

**[Table 4]**

| additive to medium | manufacturer | final concentration | recovery culture medium | activation culture medium | expansion culture medium |
|---|---|---|---|---|---|
| L-Glutamine (200 mM) | Thermo Fisher | 2 mM | + | + | + |
| Streptomycin Sulfate | MEIJI | 100 µg/mL | + | + | + |
| ITS (100x) (Insulin-Transferrin-Selenium Supplements) | Gibco | 1x | + | + | |
| Ascorbic Acid 2-Phosphate | Sigma-Aldrich | 50 µg/mL | + | + | + |
| IL-7 | Peprotech | 10 ng/mL | + | + | + |
| IL-15 | Peprotech | 10 ng/mL | + | + | + |
| IL-2 | Peprotech | 100 ng/mL | + | + | + |
| IL-18 | MBL | 50 ng/mL | | + | |
| IL-21 | Peprotech | 20 ng/mL | | + | |
| Caspase Inhibitor Z-VAD-FMK | R&D | 10 µM | | + | |
| Human CD30 antibody | R&D | 300 ng/mL | | + | |
| Human Serum Albumin 12.5 g/50 mL | CSL Behring | 2 g/L | | | + |

| | | | | | |
|---|---|---|---|---|---|
| Additives added to each group are indicated with +. | | | | | |

### 2. Reagent, antibody

RetroNectin (trade name) was purchased from Takara Bio Inc. The anti-CD3 agonist antibody used was Anti-CD3 mAb GMP grade, Anti-CD3 monoclonal antibody (Clone: OKT3) purchased from Takara Bio Inc.

### 3. Immobilization of anti-CD3 agonist antibody and RetroNectin (trade name) on culture plate

Anti-CD3 agonist antibody (OKT3, final concentration 3 µg/mL) and RetroNectin (trade name) (final concentration 150 µg/mL) dissolved in PBS at the required concentration was added to a T225 flask and left standing overnight at 4°C. After washing with PBS, the cells were subjected to the test.

### 4. Activation culture of iPS cell-derived CAR-T cell

The iPS cell-derived CAR-T cells after recovery culture were suspended at 133,333 cells/mL in IMDM medium containing 15% FBS to which the additives shown in the "activation culture medium" column in Table 4 were added, seeded into a T225 flask to which an anti-CD3 agonist antibody (OKT3) and RetroNectin (trade name) were immobilized, and cultured for 3 days under 5% CO₂/37°C.

### 5. Expansion culture of iPS cell-derived CAR-T cells in five types of serum-free media

A total of five types of expansion culture media were prepared: Advanced DMEM/F12 medium (Thermo Fisher) containing additives shown in the "expansion culture medium" column in Table 4, and said medium further added with either 0.3 mM boric acid (Sigma-Aldrich), 0.3 mM boric acid and 1×ITS-X ((Insulin-Transferrin-Selenium-Ethanolamine Supplements) Gibco), 0.3 mM boric acid and 1×ITS-X and 1 mM taurine (Sigma-Aldrich), or 0.3 mM boric acid and 1×ITS-X and 3 mM taurine (medium supplemented with only additives in Table 4, medium supplemented with additives in Table 4 + 0.3 mM boric acid, medium supplemented with additives in Table 4 + 0.3 mM boric acid + ITS-X, medium supplemented with additives in Table 4 + 0.3 mM boric acid + ITS-X + 1 mM taurine, medium supplemented with additives in Table 4 + 0.3 mM boric acid + ITS-X + 3 mM taurine). After 3 days of activation culture, the cells were collected from the T225 flask and suspended in each of the five types of expansion culture media at 40,000 cells/mL, and cultured under 5% CO₂/37°C using G-Rex (registered trademark) 10M (Wilson Wolf). Then, on the fifth day of culture, some of the cells were recovered from G-Rex (registered trademark) 10M and the cells were counted, and the medium was replaced with the respective medium. On the sixth day of culture, some of the cells were recovered from G-Rex (registered trademark) 10M and the cells were counted. On the 7th and 10th days of culture, some of the cells were recovered from the plate, counted, and seeded onto a G-Rex (registered trademark) 24-well plate (Wilson Wolf) containing each medium at 50,000 cells/mL. On the 12th and 14th days of culture, some of the cells were similarly collected from the G-Rex (registered trademark) 24-well plate, counted, and the medium was replaced with each medium.

### Experimental Example 4

### Proliferation test to verify the effect of additives of ITS-X and taurine to 0.3 mM boric acid-containing serum-free medium

In the expansion culture of item 5 in Example 4, the cell number and viability of iPS cell-derived CAR-T cells were measured on days 3, 5, 6, 7, 10, 12, and 14 of culture. The cell proliferation fold from day 0 of expansion culture is shown in Fig. 4. The proliferation of iPS cell-derived CAR-T cells was promoted by further adding 1×ITS-X and 1 mM or 3 mM taurine to 0.3 mM boric acid-containing serum-free medium (Fig. 4), and the viability was improved by 1 to 4% by adding 1 mM or 3 mM taurine to the medium (Table 5).

**[Table 5]**

| additive conditions | viability (%) | | | | | |
|---|---|---|---|---|---|---|
| | Day 5 | Day 6 | Day 7 | Day 10 | Day 12 | Day 14 |
| (1) w/o 0.3 mM Boric acid | 85.4 | 86.1 | 86.5 | 91.7 | 90.3 | 87.9 |
| (2) w/0.3 mM Boric acid | 88.7 | 88.6 | 87.5 | 90.3 | 90.4 | 88.3 |
| (3) w/0.3 mM Boric acid, ITS-X | 87.3 | 88.6 | 86.8 | 90.6 | 90.9 | 90.3 |
| (4) w/0.3 mM Boric acid, ITS-X, 1 mM Taurine | **90.2** | **90.2** | 87.6 | 91.2 | 92.1 | 91.2 |
| (5) w/0.3 mM Boric acid, ITS-X, 3 mM Taurine | 89.6 | 89.6 | **89.3** | **94.3** | **92.8** | **91.6** |
| maximum difference (%) between (3): without addition of Taurine and (4) or (5): with addition of Taurine | 2.9 | 1.6 | 2.5 | 3.7 | 1.9 | 1.3 |

| | | | | | | |
|---|---|---|---|---|---|---|
| Bold numbers indicate maximum values on each measurement date. | | | | | | |

### Example 5

### 1. Activation culture of human-derived primary T cell

Human-derived primary T cells (Leukopak-SoloPak, Charles River Laboratories Cell Solutions) were suspended at 1,000,000 cells/mL in OpTmizer medium (Thermo Fisher) containing 2% CTS Immune Cell SR (Thermo Fisher) and the additives shown in the activation culture medium in Table 6, seeded in a PL240 bag (OriGen), and cultured for 2 days under 5% CO₂/37°C.

**[Table 6]**

| additive to medium | manufacturer | final concentration | activation culture medium | transduction culture medium | expansion culture medium 1 | expansion culture medium 2 |
|---|---|---|---|---|---|---|
| OpTmizer Expansion Basal Supplement | Thermo Fisher | | + | + | + | |
| TransACT | Miltenyi Biotec | 5.4% | + | | | |
| L-Glutamine (200 mM) | Thermo Fisher | 2 mM | + | + | + | + |
| Streptomycin Sulfate | MEIJI | 100 µg/mL | + | + | + | + |
| ITS-X (100x) (Insulin-Transferrin-Selenium-Ethanol amine Supplements) | Gibco | 1x | | | | + |
| Ascorbic Acid 2-Phosphate | Sigma-Aldrich | 50 µg/mL | | | | + |
| IL-2 | Miltenyi Biotec | 40 IU/mL | + | + | + | + |
| Human Serum Albumin 12.5 g/50 mL | CSL Behring | 2 g/L | | | | + |

| | | | | | | |
|---|---|---|---|---|---|---|
| Additives added to each group are indicated with +. | | | | | | |

### 2. Reagent

RetroNectin (trade name) was purchased from Takara Bio Inc.

### 3. Immobilization of RetroNectin (product name) and retrovirus on culture plate

RetroNectin (final concentration 150 µg/mL) dissolved in PBS at the necessary concentration was added to a PL120 bag and left standing at room temperature for 2 hr. After washing with PBS, a retrovirus solution was added and immobilized overnight at 4°C while shaking at 50 rpm. After washing with PBS containing 1.5% HSA, the plate was subjected to the test.

### 4. Transduction culture of human-derived primary T cell

The human-derived primary T cells after activation culture were suspended at 722,222 cells/mL in OpTmizer medium containing 2% CTS Immune Cell SR and the additives shown in the transduction culture medium in Table 6, seeded in a PL120 bag to which RetroNectin (trade name) and a retroviral vector containing a CAR gene that recognizes a specific antigen were immobilized, and cultured for one day under 5% CO₂/37°C.

### 5. Expansion culture of human-derived primary T cell-derived CAR-T cells in four types of serum-free media

A total of four types of expansion culture media were prepared: an expansion culture medium which was OpTmizer medium containing 2% CTS Immune Cell SR and the additives shown in expansion culture medium 1 in Table 6, an expansion culture medium which was a medium containing the component shown in Table 7 (hereinafter to be referred to as BM220720) and the additives shown in expansion culture medium 2 in Table 6, said medium further added with 0.3 mM boric acid (Sigma-Aldrich), and said medium further added with 0.3 mM boric acid and 3 mM taurine (Sigma-Aldrich) (OpTmizer medium containing only additives in Table 6, and BM220720 medium containing either (i) only additives in Table 6, (ii) additives in Table 6 + 0.3 mM boric acid, or (iii) additives in Table 6 + 0.3 mM boric acid and 3 mM taurine). After one day of transduction culture, the cells were collected from the PL120 bag and suspended in each of the expansion culture media at 220,000 cells/mL, and cultured under 5% CO₂/37°C using G-Rex (registered trademark) 6M (Wilson Wolf). Then, on the seventh day of culture, some of the cells were recovered from G-Rex (registered trademark) 6M and the cells were counted, and 40 IU of IL-2 was added to each medium. On the ninth day of culture, some of the cells were recovered from G-Rex (registered trademark) 6M and the cells were counted, and seeded at 220,000 cells/mL in fresh G-Rex (registered trade mark) 6M containing respective medium. Similarly on the thirteenth and fourteenth days, some of the cells were recovered from G-Rex (registered trademark) 6M and the cells were counted.

**[Table 7]**

| component name | concentration (mg/L) |
|---|---|
| Glycine | 30 |
| L-Alanine | 25 |
| L-Arginine hydrochloride | 84 |
| L-Asparagine (freebase) | 25 |
| L-Aspartic acid | 30 |
| L-Cystine 2HCl | 91.4 |
| L-Glutamic Acid | 75 |
| L-Histidine hydrochloride-H₂O | 42 |
| L-Isoleucine | 105 |
| L-Leucine | 105 |
| L-Lysine hydrochloride | 146 |
| L-Methionine | 30 |
| L-Phenylalanine | 66 |
| L-Proline | 40 |
| L-Serine | 42 |
| L-Threonine | 95 |
| L-Tryptophan | 16 |
| L-Tyrosine 2Na 2H₂O | 121 |
| L-Valine | 94 |
| L-Ascorbic Acid 2-Phosphate Sesquimagnesium Salt | 2.5 |
| Biotin | 0.013 |
| Choline chloride | 8.98 |
| D-Calcium pantothenate | 4 |
| Folic Acid | 4 |
| Niacinamide | 4 |
| Pyridoxine hydrochloride | 2.013 |
| Riboflavin | 0.4 |
| Thiamine hydrochloride | 4 |
| Vitamin B12 | 0.68 |
| i-Inositol | 12.6 |
| Calcium Chloride (CaCl₂) (anhyd.) | 165 |
| Cupric sulfate (CuSO₄ 5H₂O) | 0.0013 |
| Ferric Nitrate (Fe(NO₃)₃9H₂O) | 0.05 |
| Ferric sulfate (FeSO₄ 7H₂O) | 0.417 |
| Magnesium Sulfate (MgSO₄) (anhyd.) | 97.67 |
| Potassium Chloride (KCl) | 330 |
| Sodium Bicarbonate (NaHCO₃) | 3024 |
| Sodium Chloride (NaCl) | 4350 |
| Sodium Phosphate monobasic (NaH₂PO₄ H₂O) | 125 |
| Zinc sulfate (ZnSO₄ 7H₂O) | 0.864 |
| Sodium Selenite | 0.005 |
| D-Glucose (Dextrose) | 4500 |
| HEPES | 5958 |
| Ethanolamine | 1.9 |
| Hypoxanthine Na | 2.39 |
| Linoleic Acid | 0.042 |
| Lipoic Acid | 0.105 |
| Putrescine 2HCl | 0.081 |
| Sodium Pyruvate | 110 |
| Thymidine | 0.365 |
| L-Glutathione reduced | 1 |
| Ammonium Metavanadate | 0.0003 |
| Manganous Chloride tetrahydrate (MnCl₂ 4H₂O) | 0.00005 |

### Experimental Example 5

### Proliferation test to verify effect of adding boric acid and taurine to human-derived primary T cell-derived CAR-T cell

To verify the effect of adding boric acid and taurine to serum-free expansion culture medium on different cell type (human-derived primary T cells), the cell number and viability of human-derived primary T cell-derived CAR-T cells were measured on days 4, 6, 10, and 11 in the expansion culture described in item 5 of Example 5. The cell proliferation fold from day 0 of expansion culture is shown in Fig. 5. The proliferation of human-derived primary T cell-derived CAR-T cells was promoted by adding 0.3 mM boric acid, or 0.3 mM boric acid and 3 mM taurine, to a serum-free medium (Fig. 5).

### Example 6

### 1. Preparation of iPS cell-derived NK cell

The iPS cell line FfI01s04 provided by the Center for iPS Cell Research and Application (CiRA), Kyoto University, was differentiated into NK cells according to a known method (e.g., the method described in WO2021/174004). The obtained cells were frozen and stored in liquid nitrogen and used as iPS cell-derived NK cells.

### 2. Recovery culture of iPS cell-derived NK cell

iPS cell-derived NK cells were suspended in a recovery culture medium, which was IMDM medium (Thermo Fisher Scientific) containing 15% Fetal Bovine Serum (FBS) and the additives shown in Table 8, at 1.25×10⁵ cells/mL, seeded in a G-Rex (registered trademark) 6-well plate (Wilson Wolf), and cultured for 3 days in a 5% CO₂/37°C incubator (days 0 to 3 of culture).

**[Table 8]**

| additive to medium | manufacturer | final concentration |
|---|---|---|
| L-Glutamine-Penicilline-Streptomycin solution | Sigma Aldrich | 1% |
| ITS(100x) (Insulin-Transferrin-Selenium Supplements) | Gibco | 1× |
| Ascorbic Acid 2-Phosphate | Sigma Aldrich | 50 µg/mL |
| IL-2 | Peprotech | 10 ng/mL |
| IL-7 | Peprotech | 10 ng/mL |
| IL-15 | Peprotech | 10 ng/mL |

### 3. Activation culture of iPS cell-derived NK cell

After recovery culture, the iPS cell-derived NK cells were suspended in an activation culture medium, which was IMDM medium (Thermo Fisher Scientific) containing 15% Fetal Bovine Serum (FBS) and the additives shown in Table 9, at 1.84×10⁵ cells/mL, seeded in a 6-well plate (TPP), and cultured for 3 days in a 5% CO₂/37°C incubator (days 3 to 6 of culture).

**[Table 9]**

| additive to medium | manufacturer | final concentration |
|---|---|---|
| L-Glutamine-Penicilline-Streptomycin solution | Sigma Aldrich | 1% |
| ITS (100x) (Insulin-Transferrin-Selenium Supplements) | Gibco | 1× |
| Ascorbic Acid 2-Phosphate | Sigma Aldrich | 50 µg/mL |
| IL-2 | Peprotech | 10 ng/mL |
| IL-7 | Peprotech | 10 ng/mL |
| IL-12 | Proteintech Group | 50 ng/mL |
| IL-15 | Peprotech | 10 ng/mL |
| IL-18 | MBL | 50 ng/mL |
| IL-21 | Peprotech | 20 ng/mL |
| Human agonistic Antibody | R&D Systems | 300 ng/mL |
| Pan Caspase Inhibitor Z-VAD-FMK | R&D Systems | 10 µM |

### 4. Expansion culture of iPS cell-derived NK cell in five types of serum-free media

After 3 days, a total of five types of expansion culture media were produced by adding 2 g/L human serum albumin (Albuminer (trade name), CSL Behring) to BM220720 medium in Table 7, further adding, to said basal medium, 0.3 mM boric acid (Sigma Aldrich), 0.3 mM boric acid + 1 mM taurine (Sigma Aldrich), and 0.3 mM boric acid + 3 mM taurine to give four types of basal media, and one type of X-VIVO (trademark) 15 medium (Lonza). To these five types of basal media were added the additives shown in Table 9 to give five types of expansion culture media. The cells that had been activation cultured for three days were suspended in each of the five types of expansion culture media at 1.25×10⁵ cells/mL, reseeded into G-Rex (registered trademark) 24-well plates (Wilson Wolf), and cultured in a 5% CO₂/37°C incubator for two days (days 6 to 8 of culture).

After two days, the media were replaced with the five types of basal media supplemented with the additives shown in Table 10, and the cells were cultured for two days in a 5% CO₂/37°C incubator (days 8 to 10 of culture).

Thereafter, the cells were reseeded in the same medium every 2-3 days onto a G-Rex (registered trademark) 24-well plate (Wilson Wolf) at 1.25×10⁵ cells/mL, and culture was continued in a 5% CO₂/37°C incubator until day 17 of culture.

**[Table 10]**

| additive to medium | manufacturer | final concentration |
|---|---|---|
| L-Glutamine-Penicilline-Streptomycin solution | Sigma Aldrich | 1% |
| ITS(100x) (Insulin-Transferrin-Selenium Supplements) | Gibco | 1× |
| Ascorbic Acid 2-Phosphate | Sigma Aldrich | 50 µg/mL |
| IL-2 | Peprotech | 10 ng/mL |
| IL-7 | Peprotech | 10 ng/mL |
| IL-15 | Peprotech | 10 ng/mL |
| IL-21 | Peprotech | 20 ng/mL |

### Experimental Example 6

### Proliferation test to verify the effect of additives of boric acid and taurine on iPS cell-derived NK cell

To verify the effect of adding boric acid and taurine to serum-free expansion culture medium on iPS cell-derived NK cells, the cell number and viability of iPS cell-derived NK cells were measured over time during the expansion culture in item 4 of Example 6. The proliferation fold and cell viability on day 11 of expansion culture are shown in Table 11. The proliferation of iPS cell-derived NK cells was promoted by adding 0.3 mM boric acid and 1 mM taurine or 3 mM taurine to a serum-free basal medium (Table 11).

**[Table 11]**

| basal medium used for expansion culture | proliferation fold for 11 days | cell viability (%) on 11th day of expansion culture |
|---|---|---|
| X-VIVO-15 | 71 | 97.4 |
| BM220720+2 g/L HSA | 210 | 97.3 |
| BM220720+2 g/L HSA+0.3 mM Boric acid | 212 | 96.8 |
| BM220720+2 g/L HSA+0.3 mM Boric acid+1 mM Taurine | 238 | 97.2 |
| BM220720+2 g/L HSA+0.3 mM Boric acid+3 mM Taurine | 253 | 96.7 |

### [Industrial Applicability]

According to the present invention, a cell proliferation maintenance-promotion effect comparable to that by culture in a serum-containing medium can be obtained even when a serum-free medium or low-serum medium is used. In particular, culture in a serum-free medium or low-serum medium that does not require extensive washing is preferred for cells that are vulnerable to a stress caused by operations such as washing of cell and the like, and the present invention is suitable for culture under such conditions.

This application is based on a patent application No. 2022-102986 filed in Japan (filing date: June 27, 2022), the contents of which are incorporated in full herein.

## Claims

1. A method for culturing a cell, comprising a step of culturing the cell in a serum-free medium or low-serum medium comprising boric acid or a salt thereof.

2. The method according to claim 1, wherein the final concentration of the boric acid or a salt thereof in the medium is 0.1 to 0.9 mM.

3. The method according to claim 1, wherein the final concentration of the boric acid or a salt thereof in the medium is 0.3 to 0.6 mM.

4. The method according to claim 1, wherein the serum-free medium or low-serum medium is a serum-free medium.

5. The method according to claim 1, wherein the cell is an immunocompetent cell.

6. The method according to claim 5, wherein the immunocompetent cell is selected from dendritic cell, B cell, T cell, and natural killer cell.

7. The method according to claim 6, wherein the immunocompetent cell is a T cell.

8. The method according to claim 1, wherein the cell is derived from a pluripotent stem cell.

9. The method according to claim 8, wherein the pluripotent stem cell is an iPS cell.

10. The method according to claim 1, wherein the medium further comprises taurine.

11. A method for proliferating a cell, comprising a step of culturing the cell in a serum-free medium or low-serum medium comprising boric acid or a salt thereof.

12. The method according to claim 11, wherein the serum-free medium or low-serum medium is a serum-free medium.

13. The method according to claim 11, wherein the medium further comprises taurine.

14. A method for producing a cell population in which the number of desired cells is expanded, comprising a step of culturing a cell population comprising the desired cells in a serum-free medium or low-serum medium comprising boric acid or a salt thereof.

15. The method according to claim 14, wherein the serum-free medium or low-serum medium is a serum-free medium.

16. The method according to claim 14, wherein the medium further comprises taurine.

17. A medicament comprising a cell population obtained by the method according to claim 14.

18. The medicament according to claim 17, which is for use in the prophylaxis and/or treatment of cancer.

19. A medium additive comprising boric acid or a salt thereof, wherein the medium is a serum-free medium or a low-serum medium.

20. The additive according to claim 19, wherein the serum-free medium or low-serum medium is a serum-free medium.

21. The additive according to claim 19, wherein the medium further comprises taurine.

22. A medium composition comprising boric acid or a salt thereof, wherein the composition is obtained by adding the medium additive according to claim 19 to a serum-free medium or low-serum medium.

23. The medium composition according to claim 22, wherein the serum-free medium or low-serum medium is a serum-free medium.

24. The medium composition according to claim 22, further comprising taurine.

25. A method for the prophylaxis and/or treatment of cancer, comprising administering a cell population obtained by the method according to claim 14 to a subject in need thereof.

26. A cell population obtained by the method according to claim 14, which is for use in the prophylaxis and/or treatment of cancer.

27. Use of a cell population obtained by the method according to claim 14 in the production of a medicament for the prophylaxis and/or treatment of cancer.
